# EUROPEAN PATENT APPLICATION

(11) **EP 4 403 110 A1**
(43) Date of publication of application: **24.07.2024**
(21) Application number: 22870123.1
(22) Date of filing: 20.07.2022
(51) Int. Cl.: A61B 6/00, G06T 7/00, G06T 5/00

(54) **IMAGE PROCESSING METHOD AND APPARATUS**

(30) Priority: 14.09.2021 KR 20210122696
(71) Applicant: LG Electronics Inc., Yeongdeungpo-gu Seoul 07336 (KR)
(72) Inventor: KIM, Sunghyun, Seoul 06772 (KR); HONG, Yunpyo, Seoul 06772 (KR); IM, Hyewon, Seoul 06772 (KR); YOO, Jongsang, Seoul 06772 (KR)
(74) Representative: Vossius & Partner Patentanwälte Rechtsanwälte mbB
(86) International application number: PCT/KR2022/010640
(87) International publication number: WO 2023/043036

(57) **Abstract**

Disclosed are an image processing method and device. Here, the X-ray image processing device according to an embodiment of the present disclosure includes an image analyzer that analyzes the received original X-ray image; A conversion unit that converts the original X-ray image into multi-scale based on the analysis results and separates it into frequency units; A noise prediction unit that generates a noise prediction map by enhancing and combining specific frequency portions; an edge map processor that generates an edge map based on the generated noise prediction map; A contrast processing unit that enhances the contrast of the X-ray image based on the generated edge map; an inverse transformation unit that performs a flattening process and an edge correction process on the contrast-enhanced X-ray image and performs inverse transformation; and a control unit that tone maps the inversely converted X-ray image and controls output.

## Description

### [Technical field]

The present disclosure relates to image processing, and more specifically, to an image processing method and device that reduces a radiation exposure dose or, regardless, processes the image quality deterioration of images acquired through radiation to be minimized.

### [Background]

As medical devices and related technologies develop, the medical device market is growing rapidly. Among these, in particular, X-ray medical devices are currently the most widely distributed and in high demand.

These X-ray medical devices are devices that image the inside of the human body for radiological examination.

However, images obtained from X-ray medical devices, that is, X-ray images, have uneven brightness levels and are difficult to distinguish by region, making precise diagnosis or accurate judgment of the X-ray images difficult.

In addition, X-ray images have a wide dynamic range of 16 bits or more in general, and information on the region of interest for the X-ray images is concentrated in a narrow area, making it difficult to apply existing processing methods to general images.

Accordingly, there have been attempts to provide high-definition X-ray images to enable more precise diagnosis and accurate judgment of conventional X-ray images, but there are still difficult to obtain high-quality X-ray images due to issues with processing artifacts such as noise and contrast processing.

Meanwhile, to obtain high-quality images through X-ray medical devices and increase the accuracy of diagnosis, images must be taken under high-dose conditions, but there is a problem the higher the dose, the more radiation exposure to the human body increases.

Accordingly, there is a need for a method of obtaining X-ray images without problems with image quality for diagnosis while minimizing radiation exposure by taking images with the minimum dose.

### [Detailed Description of the Invention]

### [Technical problem]

The purpose of the present disclosure is to provide an image processing method and device for processing X-ray images taken with a minimum dose to prevent or minimize image quality deterioration.

Another purpose of the present disclosure is to provide an image processing method and device for processing to prevent or minimize image quality deterioration of X-ray images obtained regardless of the radiation dose.

Another purpose of the present disclosure is to provide image processing methods and devices that prevent or minimize image quality deterioration by processing the obtained X-ray image by considering the noise characteristics of the boosted image according to the body part being imaged, shooting conditions, etc.

### [Technical solution]

An X-ray image processing device according to an embodiment of the present disclosure can include an image analyzer configured to analyze a received original X-ray image; a converter configured to convert the original X-ray image into multi-scale based on the analysis results and separate the converted original X-ray image into frequency units; a noise predictor configured to generate a noise prediction map by enhancing and combining specific frequency portions; an edge map processor configured to generate an edge map based on the generated noise prediction map; a contrast processor configured to enhance the contrast of the X-ray image based on the generated edge map; an inverse converter configured to perform a flattening process and an edge correction process on the contrast-enhanced X-ray image and perform an inverse conversion to the X-ray image; and a controller configured to perform a tone-map to the inversely converted X-ray image and control the tone-mapped X-ray image to be output.

A method of processing an X-ray image according to an embodiment of the present disclosure can include receiving an original X-ray image; analyzing the received original X-ray image; converting the original X-ray image into a multi-scale based on the analysis results and separating the multi-scale into frequency units; generating a noise prediction map by enhancing and combining specific frequency portions; generating an edge map based on the generated noise prediction map; enhancing a contrast of the X-ray image based on the generated edge map; performing a flattening process and an edge correction process on the contrast-enhanced X-ray image and inversely converting the X-ray image; and tone mapping and outputting the inversely converted X-ray image.

### [Effects of the Invention]

According to an embodiment of the present disclosure, it is possible to obtain image quality, that is similar to standard dose, suitable for diagnosis while reducing radiation exposure to the human body by processing noise, contrast, etc. for low-dose X-ray images.

According to an embodiment of the present disclosure, noise processing is primarily performed on the X-ray image converted to multi-scale through various processes, and noise processing is additionally performed in the inverse conversion process to improve the accuracy of noise removal. It has the effect of not only increasing the image quality but also preventing the resulting deterioration in image quality.

According to an embodiment of the present disclosure, there is an effect of providing images with consistent image quality regardless of the radiation dose.

### [Brief description of the drawing ]

Figure 1 is a schematic diagram of an image processing system according to an embodiment of the present invention.
Figure 2 illustrates an example of the image processing system of Figure 1 is actually implemented.
Figure 3 is a block diagram of an image processing device according to an embodiment of the present invention.
Figure 4 is a flowchart that explains an image processing method according to an embodiment of the present invention.
Figure 5 is a diagram illustrating image analysis according to an embodiment of the present invention.
Figures 6 and 7 are diagrams to explain noise prediction according to an embodiment of the present invention.
Figure 8 is a diagram illustrating edge map generation according to an embodiment of the present invention.
Figure 9 is a diagram illustrating range control according to an embodiment of the present invention.
Figure 10 is a diagram illustrating inverse transformation according to an embodiment of the present invention.
Figure 11 is a diagram illustrating inverse transformation according to another embodiment of the present invention.
Figure 12 is a diagram to explain noise and contrast control according to an embodiment of the present invention.
Figure 13 is a diagram illustrating an original image and a processed image according to an embodiment of the present invention.

### [Best mode]

Hereinafter, embodiments related to the present invention will be described in more detail with reference to the drawings. The suffixes "module" and "part" for components used in the following description are given or used interchangeably only for the ease of preparing the specification and do not have distinct meanings or roles in themselves.

An image processing method and device according to the present disclosure are disclosed.

In the present disclosure, a noise map is generated by predicting noise characteristics for each brightness of an X-ray image, and a more robust edge map is generated by utilizing various information such as edge detection, standard deviation, a correlation between layers.

In addition, in the present disclosure, unlike the prior art, not only is noise suppressed for each layer separated by frequency through multi-scale conversion, but also noise that may occur in the process of combining frequencies during later inverse conversion is removed, and performing edge correction considering the directionality of the edge. Thus, the noise included in the X-ray image can be minimized by double processing the noise and improving the accuracy of noise removal.

Therefore, according to the present disclosure, when removing noise, noise prediction information, edge strength, characteristics of each target region, etc. are together taken into consideration, so it is possible to derive results that are more adaptive to a signal, and can provide consistent image quality correction even in low-dose X-ray images or dose-independent, that is high-dose or standard dose.

'Images' described in the specification refers to radiation, particularly X-ray images obtained from an X-ray machine, but are not limited thereto.

With reference to the accompanying drawings, the image processing method according to the present disclosure, in particular, describes a method of preventing or minimizing image quality degradation while reducing the radiation dose to the human body (e.g., bones of the subject of imaging, etc.) through low-dose X-rays as well as standard doses.

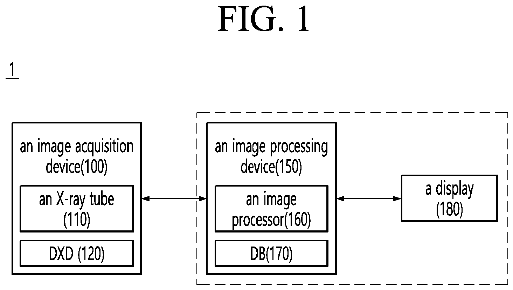

Figure 1 is a schematic diagram of an image processing system 1 according to an embodiment of the present invention.

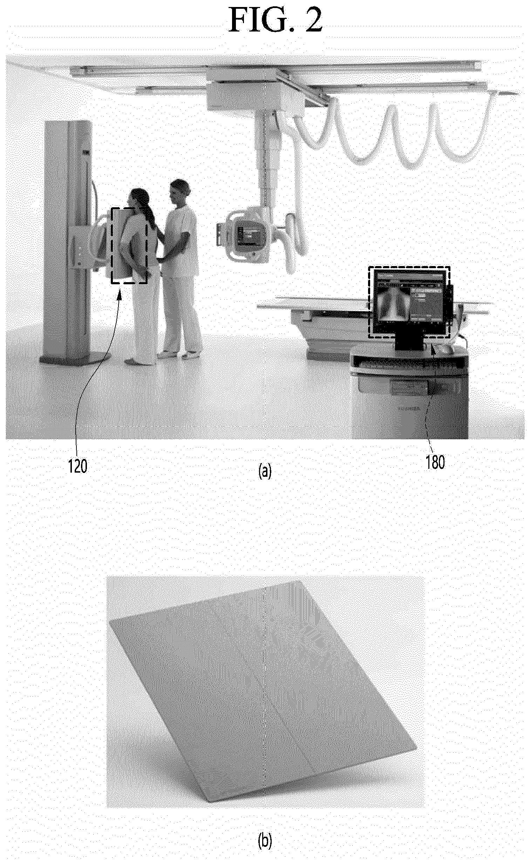

FIG. 2 illustrates an example of the image processing system 1 of FIG. 1 is actually implemented.

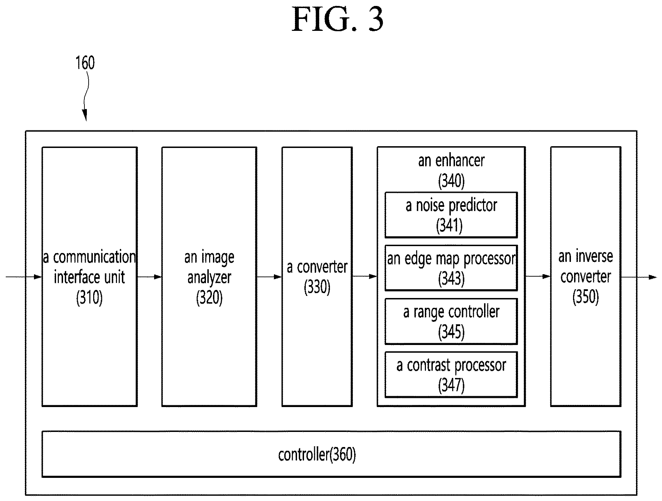

Figure 3 is a block diagram of the image processing device 150 according to an embodiment of the present invention.

Referring to FIG. 1, the image processing system 1 may largely include an image acquisition device 100, an image processing device 150, and an image output device 180.

Depending on the embodiment, the image output device 180 may be a component of the image processing device 150.

The image acquisition device 100 may include an X-ray tube 110 and a digital X-ray detector (DXD) 120.

The X-ray tube 110 irradiates a certain amount of X-rays to a subject (e.g., a body part).

The digital X-ray detector 120 detects an X-ray image based on X-rays irradiated to the subject.

The image acquisition device 100 may transmit the image detected through the digital X-ray detector 120 to the image processing device 150.

In this case, the image acquisition device 100 and the image processing device 150 can perform data communication through a wired/wireless network.

Next, the image processing device 150 can process the X-ray image received through the image acquisition device 100 and provide the processed X-ray image through the display 180.

When an X-ray image is input, the image processing device 150 according to an embodiment of the present disclosure can segment a background region, an anatomy region, a collimation region, and a metal object, etc. from the input X-ray image, store information on that, divide the segmented regions by multi-frequency (i.e., frequency unit). The image processing device 150 can first perform edge map generation, noise suppression for each layer and/or area and contrast boost using generated noise prediction map by frequency (or brightness), predicted noise information, the correlation between image analysis information between layers, by utilizing image analysis information, and then secondarily, during the layer combining (inverse transformation) process, apply additional noise removal and edge correction considering brightness (thickness of the object), additional noise removal and edge directionality (e.g., in specific frequency units or specific layers), thereby being not only a more natural image quality but also a consistency of image quality, that is, performing contrast automatic control to maintain consistency between images.

Referring to FIG. 3, the image processor 160 can be implemented by including a communication interface unit 310, an image analyzer 320, a converter 330, an enhancer 340, an inverse converter 350, and a controller 360 and the like.

Depending on the embodiment, some of the components as shown in FIG. 3 may be omitted or modularized together with other components, and components as not shown in FIG. 3 may be added.

The communication interface unit 301 can provide an interfacing environment for data communication with the image acquisition device 100, and receive X-ray images obtained from the image acquisition device 100.

The communication interface unit 301 may perform short-range communication with the image acquisition device 100. To this end, the communication interface unit 301 can support short-range communication using at least one technology among Bluetooth^{™}, Bluetooth Low Energy (BLE), Radio Frequency Identification (RFID), Infrared Data Association (IrDA), Ultra Wideband (UWB), ZigBee, and NFC (Near Field Communication), Wi-Fi (Wireless-Fidelity), Wi-Fi Direct, and Wireless USB (Wireless Universal Serial Bus). This communication interface unit 310 can support wireless communication between the image acquisition device 100 and the wireless communication system, between the image processing device 150 and the display 180, or between the image processing device 150 and the network where the server is located.

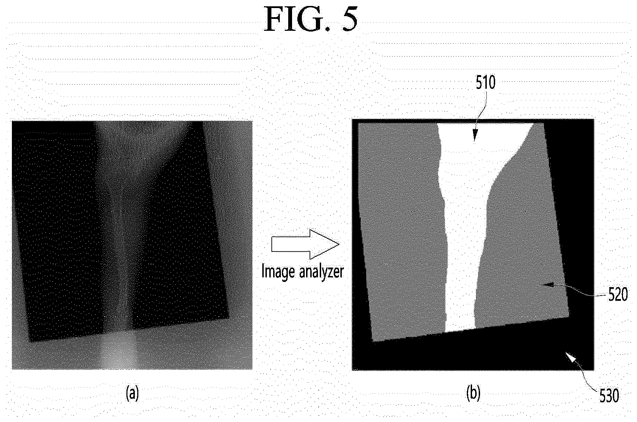

The image analyzer 320 can perform analysis on the X-ray image received through the communication interface unit 310. In this case, the image analysis can be referred to as predicting and dividing an anatomy region 510, a direct exposure region 520, and a collimation region 530, as shown in (b) of FIG. 5, and among the divided regions, in particular, noise and edges can be separated for the anatomy region 510. By separating noise and edges through the image analysis, it is possible to control noise and edges not to be enhanced together. That is, the image processing device 150 can perform first processing by suppressing the separated noise and more emphasizing the edges.

The converter 330 can perform multi-scale transformation to the original X-ray image data or raw X-ray image data received through the communication interface unit 310 to be enhanced in frequency unit characteristics.

Depending on the embodiment, the converter 330 may perform a log transformation on the original X-ray image before performing the multi-scale transformation.

The enhancer 340 can include a noise predictor 341, an edge map processor 343, a range controller 345, a contrast processor 347, etc.

Depending on the embodiment, the converter 330 and the enhancer 340 can be modularized.

Depending on the embodiment, the enhancer 340 may be implemented in the form of a plurality of modules.

The results of image analysis through the image analyzer 320 can be transferred to and utilized by at least one component of enhancer 340.

The noise predictor 341 can generate noise prediction information from the multi-scale transformed X-ray image based on the analysis results of the original X-ray image.

The edge map processor 343 can generate an edge map based on the analysis results of the original X-ray.

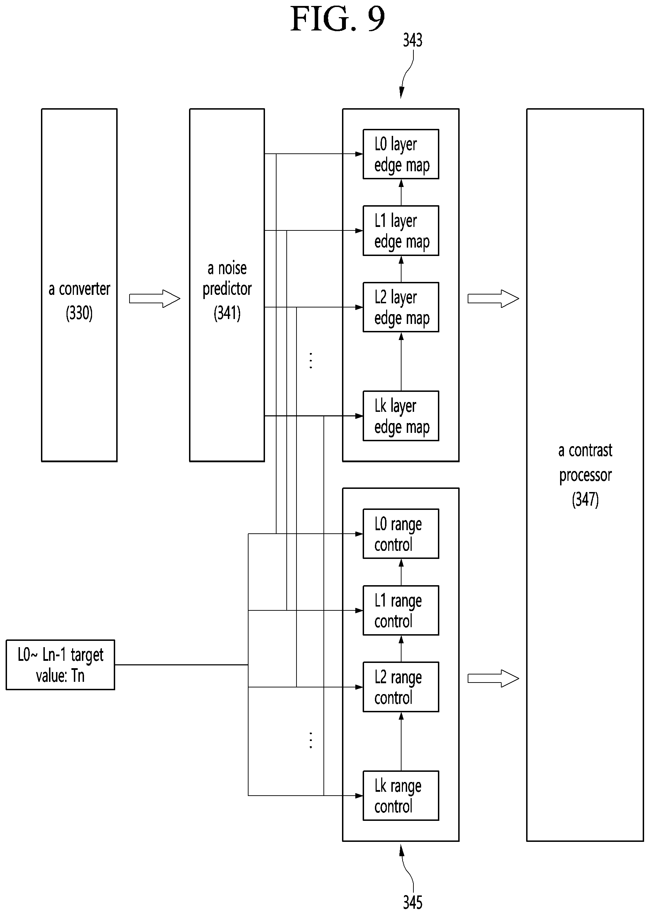

The range controller 345 can perform range control for each layer for the multi-scale transformed X-ray image based on the analysis results of the original X-ray image and the noise prediction information generated by the noise predictor 341. Here, the range control can be referred to, for example, as related to the consistency of image quality, and as shown in FIG. 9, contrast normalization in certain ranges where the resultant is derived using a reference value. This can be seen as an operation that enables consistent image quality processing regardless of dose.

This range control can be adjusted the standard deviation for each layer and image.

Meanwhile, as shown in FIG. 9, the reference value can represent a reference value according to the characteristics of each layer, and the reference value can be different for each layer. Additionally, these reference values can be values calculated in advance through experiments, etc.

The contrast processor 347 can perform detailed contrast enhancement through controlling base layer contrast and noise based on the analysis results of original X-ray image.

The inverse converter 350 can process inverse transformation to the X-ray image processed through the contrast processor 347.

In addition, the controller 360 can control the operation of the image processor 160. Accordingly, the controller 360 can appropriately control the operation of each component of the image processor 160 through data communication with each component of the image processor 160. This controller 360 can control processed data such as received, generated, etc. through the data communication to be stored in the DB 170.

The database (DB) 170 can temporarily store received X-ray images. Unlike shown in FIG. 1, the database (DB) 170 does not necessarily need to be built into the image processing device 150 and can be located externally or remotely.

Referring to FIGS. 1 to 3, the display 180 may receive and output the X-ray image processed through the image processor 160. In this case, the display 180 can be in the form of a fixed device such as a monitor, a TV, or a signage, or a mobile device such as a cell phone, a tablet PC, or a laptop. Depending on the embodiment, the display 180 can be a dedicated device for outputting image according to the present disclosure.

Here, the display 180 can be a wearable device (e.g., a smartwatch, smart glasses) and a mobile terminal such as a smartphone capable of exchanging data with (or interoperating with) another display device (not shown).

The communication interface unit 310 can detect (or recognize) a wearable device capable of communication around the image processing device 150.

Furthermore, if the detected wearable device is an authenticated device to communicate with the image processing device 150 according to the present invention, the controller 340 can control at least part of data processed by the image processing device 150 to be transmitted to the wearable device through the communication interface unit 301.

Accordingly, a user of the wearable device can use the data processed on the display 180 through the wearable device.

Meanwhile, the image processing system 1, the image acquisition device 100, or the image processing device 150 as shown in FIGS. 1 and 3, which are only an example of the present disclosure, some of the illustrated components may be integrated, added, or omitted depending on the specifications of the actually implemented system or device.

In other words, as needed, two or more components can be combined into one component, or conversely, one component can be subdivided into two or more components. In addition, the functions performed by each component are for explaining embodiments of the present invention, and the specific operations or devices do not limit the scope of the present invention.

The operation of the image processing system 1 of FIGS. 1 to 3 as described above will be described in more detail referring to the attached drawings as follows.

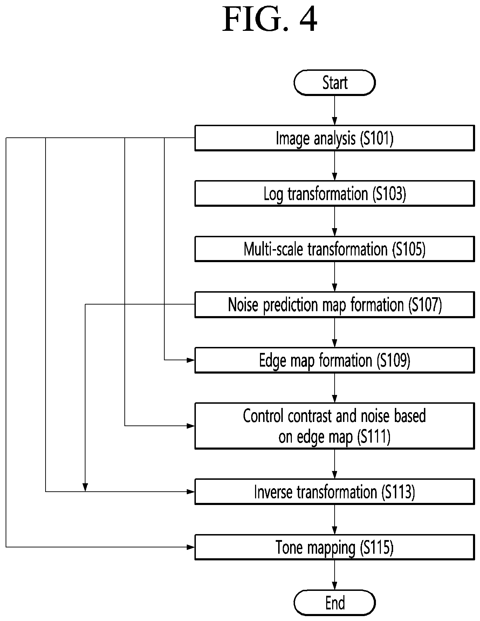

Figure 4 is a flowchart illustrating an image processing method according to an embodiment of the present invention.

Hereinafter, FIG. 4 is described from the perspective of the image processing device 150 but is not limited thereto.

The image processing device 150 can analyze the original X-ray image obtained from the image acquisition device 100 through the communication interface unit 301 (S101).

The image processing device 150 can segment the original X-ray image into the anatomy region 510, the direct exposure region 520, and the collimation region 530, as shown in (b) of Figure 5, the anatomy area 510 among the segmented regions can be analyzed and the analysis results can be stored.

That is, the image processing device 150 can predict the anatomy region 510, the direct exposure region 520 and the collimation region 530 by applying segmentation technology to the original X-ray image and generate a map, and then enhancement is performed only for the desired region (e.g., only for the edge region for the anatomy region 510).

The image processing device 150 can perform a first conversion on the original X-ray image (S103). In this case, the first conversion can mean, for example, log transformation, but is not limited thereto.

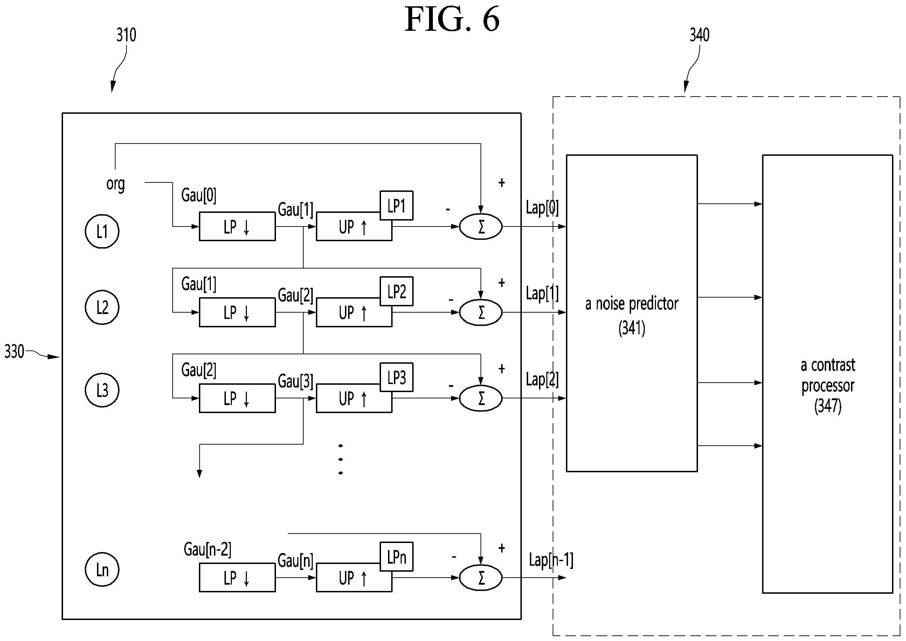

The image processing device 150 can perform a second conversion process on the first converted X-ray image (S105). Here, the second transformation process is, for example, as shown in FIG. 6, a multi-scale transformation in which the original X-ray image is separated into n layers in frequency units, as an example, but the present disclosure is not limited to thereto.

The image processing device 150 can generate a noise prediction map from the second converted X-ray image.

The image processing device 150 can use a Gaussian-Laplacian pyramid structure as shown in FIG. 6 for multi-scale conversion.

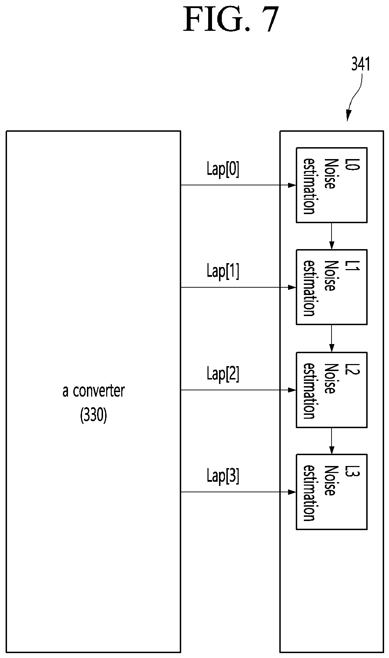

Additionally, the image processing device 150 can generate the noise prediction map as shown in FIGS. 6 and 7.

In relation to the present disclosure, due to the nature of the X-ray image, the points requiring processing or improvement may be different depending on the target area, target region, etc.

Therefore, the image processing device 150 according to an embodiment of the present disclosure can obtain an appropriate image suitable to a target area by dividing it into frequency units based on multi-scale (or multi-frequency) and adjusting parameters for each frequency. As described above, the present disclosure uses a Gaussian-Laplacian pyramid that can be used as a multi-scale transformation (or multi-frequency transformation) method but is not limited thereto.

Regarding the noise prediction, for example, an X-ray image can be converted to a root square. However, when converting the X-ray image into a root square, global contrast at a low signal level can deteriorate.

Accordingly, in the present disclosure, the above-described log transformation is used as an example but is not limited thereto. On the other hand, in the case of the log transformation, since the deviation of the noise changes depending on the intensity, in the present disclosure, the deviation of the noise can be predicted for each brightness and utilized as a brightness variable prediction function.

In the present disclosure, when predicting noise for each layer, the noise in other layers can be corrected based on the predicted information in the layer that has the greatest impact on the noise, rather than predicting it independently for each layer.

First, the converter 330 can adopt the Gaussian-Laplacian pyramid structure as shown in FIG. 6 for multi-scale conversion processing, but is not limited to thereto.

Referring to FIG. 6, the converter 330 can form n layers (where n is a natural number), that is, a multi-layer structure, with reference to the original X-ray image.

The n can be determined according to the settings of the image processing device 150. For example, if n is 10, a total of 10 images, that is, L1 - L10, can be processed.

In this case, the smaller n is, the closer it is to the original X-ray image, and it may contain more noise than when n is relatively large. Therefore, in the present disclosure, noise prediction (or estimation) can be made using Gaussian information corresponding to the brightness in the layer corresponding to the high-frequency portions, that is, L1 to L3.

Referring to FIG. 7, the noise predictor 341 is shown to include four noise prediction modules that each can predict noise, but the number of modules is not limited to what is shown. That is, the number of noise prediction modules included in the noise predictor 341 may be different from what is shown.

Referring to FIGS. 6 and 7, among the outputs of converter 330, the output value Lap [0] of the L1 layer is input to a first noise prediction module (L0 noise estimation), and the output value Lap [1] of the L2 layer is input to a second noise prediction module (L1 noise estimation), the output value Lap [2] of the L3 layer is input to the third noise prediction module (L2 noise estimation), and the output value Lap [3] of the L4 layer is input to the fourth noise prediction module (L3 noise estimation).

Meanwhile, noise predictor 341 can predict noises of other layers by providing the noise information to the next noise prediction module based on the noise information predicted by the first noise prediction module. In this case, the noise information predicted by the first noise prediction module is used as a reference because the input of the first noise prediction module practically contains the most noise and thus has the greatest influence on noise prediction. However, the present disclosure is not limited to thereto, and a value predicted by noise in another layer can be further referred to as a noise reference value.

The edge map processor 343 can generate an edge map based on the analysis results of the original X-ray image (S109).

The contrast processor 347 can perform detail contrast enhancement through contrast and noise control at base layer based on the analysis results of the original X-ray image (S111).

In the above, the range controller 345 can perform range control for each layer of the second converted X-ray image based on the analysis results of the original X-ray image and the noise prediction information generated by the noise predictor 341.

The image processing device 150 may form an edge map based on a multi-featured. In this case, the multi-featured can be referred to as information about noise, edge, contrast, layer, etc., but is not limited thereto.

The edge map processor 343 can predict the noise level of each Laplacian using Gaussian information, anatomy, and layer-specific information and the like corresponding to the brightness in the high-frequency layers (Layers 0 to 3 and 4).

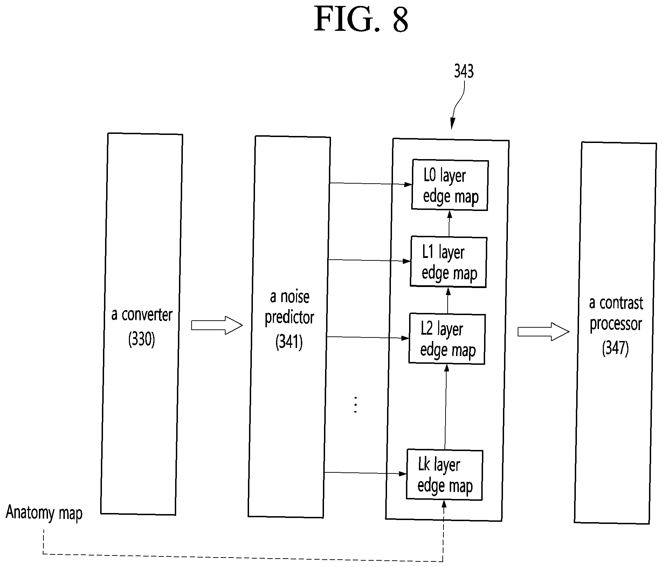

Referring to FIGS. 7 and 8, in the present disclosure, a local standard deviation value can be calculated, but when an edge map is generated only with the calculated local standard deviation value, since the directionality information of the edge is not considered, the around of the edge can be messy. Additionally, in the above case, when the image processing device 150 generates a map independently for each layer without considering the correlation between layers, noise removal can be limited in higher frequency layers.

In the present disclosure, considering the problems, in addition to the local standard deviation value as calculated above, a more robust map can be generated by reflecting local edge information and correlation between layers in a specific frequency unit.

Referring to FIGS. 7 and 8, the correlation can be performed by combining an edge map and a contrast map, and the combined value can be divided based on the noise prediction information predicted in FIG. 7. This can be, for example, to determine whether to suppress an intensity for the map according to the noise.

Referring to FIGS. 8 and 9, the image processing device 150 can perform map-based layer contrast, noise control and local contrast consistency processing.

In this case, as shown in FIG. 9, the image processing device 150 can perform range adjustment according to a predefined target reference (Tn) of the Laplacian change rate for each region and layer and adaptive Laplacian boost according to the edge map.

As shown in FIG. 8, the image processing device 150 can assign weights to the Laplacian values of each layer using an edge map.

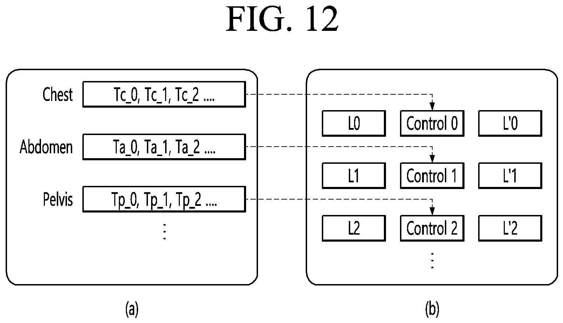

Referring to (a) of FIG. 12, the X-ray image has an optimal range for each imaging area (e.g., chest, hand, pelvis, etc.) and each frequency unit (layer 0, 1, 2, ...) of the same area (Laplacian). Accordingly, the image processing device 150 can collect X-ray images taken with the most optimal dose for each imaging area and measure an appropriate range of the Laplacian for each layer of the collected X-ray images.

In the present disclosure, for convenience, when measuring the range, only the value for the anatomy region predicted as a result of image analysis in (b) of FIG. 5 can be selectively performed rather than measuring the entire region.

As shown in (b) of FIG. 9, when an input image (X-ray image) is received with reference to the optimal range value for each region and layer obtained in advance, the range can be calculated for each layer and the weight is automatically adjusted according to a reference value (Tn of 9), and images of similar areas can produce consistent image quality.

The inverse converter 350 can perform a third conversion process on the X-ray image processed through the contrast processor 347 (S113). In this case, the third transformation process may represent, for example, inverse transformation. On the other hand, the inverse transformation may be Inverse FFT (IFFT) for responding to multi-scale transformation.

The image processing device 150 can inversely transform the multi-scale converted X-ray image, which may include processing processes such as local contrast consistency, noise reduction, and edge correction.

For example, the image processing device 150 can perform histogram-based equalization on the middle layer (intermediate frequency) for contrast consistency.

The image processing device 150 can perform a smoothing operation on signals that are not smooth due to boosting in the low layer (high frequency) while adaptively preserving edges using anatomy, brightness, edge map information, etc.

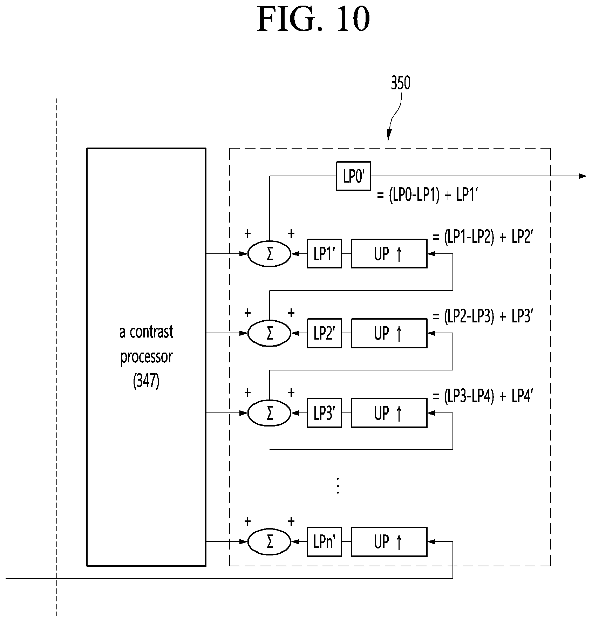

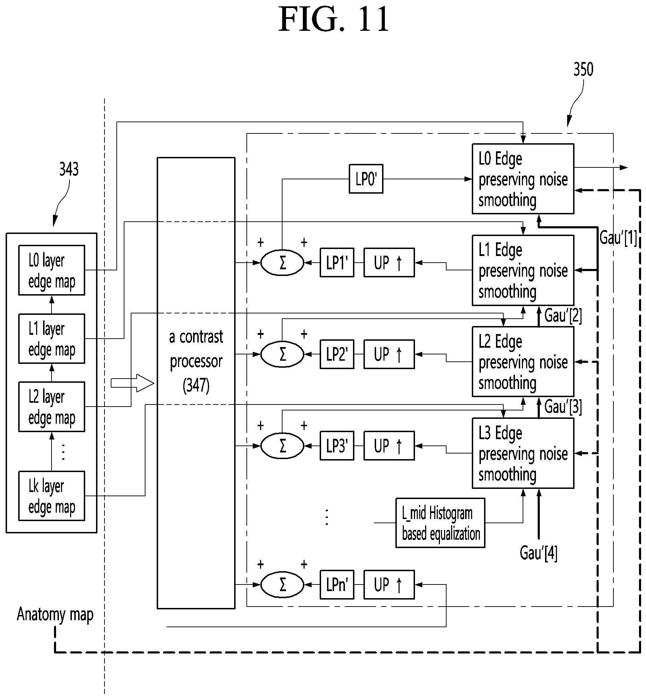

In the inverse transformation process, the inverse converter 350 can generate a final result image by adding the corrected frequency components sequentially, for example, starting from the high layer (Ln), as shown in FIG. 10 or 11.

In the inverse transformation process, even if the frequency components are boosted to add to the brightness of the X-ray image for each layer (consistency), in case the brightness is too bright or dark, the overall contrast may not be leveled in the final X-ray image. To solve this problem, the inverse transform unit 350 can flatten the brightness and contrast of the image based on the histogram in the above-described intermediate frequency layer. Through the flattening, the inverse transformer 350 not only maintains consistency of the output X-ray image regardless of the dose but also prevents noise due to high frequencies from being boosted.

As described above, even if a noise is first suppressed in transformer 330, the noise can be amplified again during a merging step for each layer during the inverse transform process. To deal with that, additional noise removal can be performed separately in layers L2, L1, and L0, which have a relatively large noise influence.

The separate noise removal may include smoothing in consideration of small-sized noise or the directionality of unnatural signals due to the above-mentioned boost operation, rather than removing relatively large-sized noise.

In addition, the controller 360 can perform output to be controlled by performing a tone mapping operation on the inversely converted X-ray image (S115). The tone mapping can indicate, for example, that the X-ray image itself is a 16-bit image and has a wide range, while the display 180 supports an 8-bit, so the range does not match, and this is corrected.

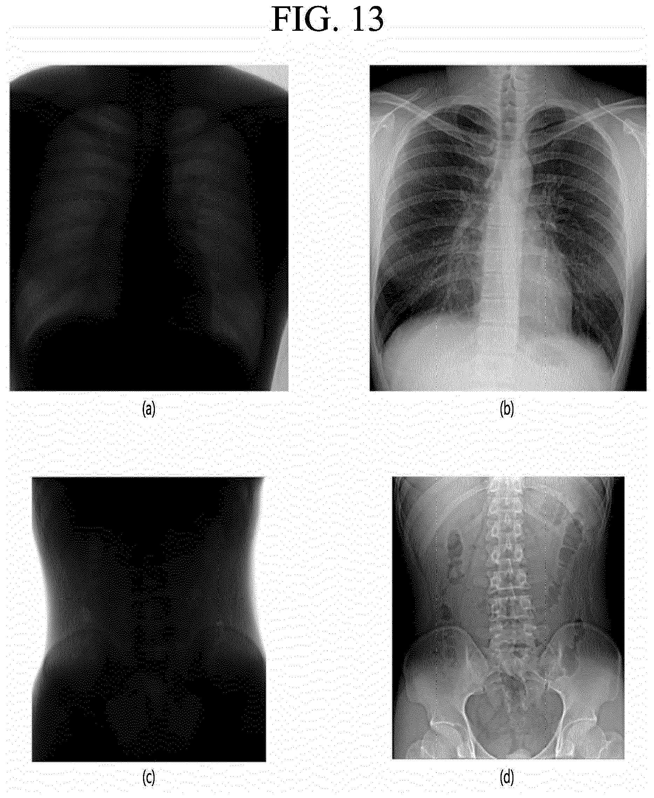

Figure 13 illustrates the original X-ray image and the post-processed X-ray image before processing according to the present invention. FIGS. (a) and (c) of 13 illustrate the original X-ray image, and FIGS. (b) and (d) of 13 illustrate the post-processed X-ray image according to the present invention described above.

Although the present specification has been described using an X-ray image as an example, it is not necessarily limited thereto. In addition, the present invention can be applied in the same or similar manner to various industrial fields that use X-rays in addition to the medical field.

The above description is merely an illustrative explanation of the technical idea of the present invention, and various modifications and variations can be made by those skilled in the art without departing from the essential characteristics of the present invention.

Accordingly, the embodiments disclosed in the present invention are not intended to limit the technical idea of the present invention, but are for illustrative purposes, and the scope of the technical idea of the present invention is not limited by these embodiments.

The scope of protection of the present invention shall be interpreted in accordance with the claims below, and all technical ideas within the equivalent scope shall be construed as being included in the scope of rights of the present invention.

### [Industrial Applicability]

According to the image processing device according to the present disclosure, it has the effect of not only minimizing image quality deterioration for low-dose X-ray image, but also ensuring or providing consistent image quality without dose, so industrial applicability of the present disclosure is significant.

## Claims

1. An X-ray image processing device, comprising:
an image analyzer configured to analyze a received original X-ray image;
a converter configured to convert the original X-ray image into multi-scale based on the analysis results and separate the converted original X-ray image into frequency units;
a noise predictor configured to generate a noise prediction map by enhancing and combining specific frequency portions;
an edge map processor configured to generate an edge map based on the generated noise prediction map;
a contrast processor configured to enhance the contrast of the X-ray image based on the generated edge map;
an inverse converter configured to perform a flattening process and an edge correction process on the contrast-enhanced X-ray image and perform an inverse conversion to the X-ray image; and
a controller configured to perform a tone-map to the inversely converted X-ray image and control the tone-mapped X-ray image to be output.

2. The X-ray image processing device of claim 1, wherein the image analyzer is configured to:
segment the received X-ray image into an anatomy region, a direct exposure region, and a collimation region,
separate a noise region and an edge region for the segmented anatomy region,
suppress the separated noise region and
boost the separated edge region.

3. The X-ray image processing device of claim 2, wherein the converter is configured to use a Gaussian-Laplacian pyramid structure.

4. The X-ray image processing device of claim 3, wherein the noise predictor is configured to:
generate noise prediction information for each layer based on information on a plurality of layers corresponding to high-frequency portions, wherein the information on the plurality of layers is based on the Laplacian value calculated using Gaussian information corresponding to the brightness of the original image and the specific frequency portions, and
correct noise prediction information for the next higher layer by reflecting the noise prediction information predicted for the highest layer among the plurality of layers.

5. The X-ray image processing device of claim 4, wherein the edge map processor is configured to:
generate an edge map for each layer corresponding to the high-frequency portions, generate a contrast map, correlate each layer corresponding to the high-frequency portions, and determine and apply whether to suppress or suppression strength for the edge map of each layer through the correlation, and
wherein, based on the anatomy region information, a combined value of the generated contrast map and the generated edge map is divided by the noise prediction information for the corresponding layer to determine whether to suppress the edge map or the suppression strength for each layer.

6. The X-ray image processing device of claim 5, further comprises:
a range controller configured to perform normalization for each layer corresponding to the high-frequency portions based on a predefined target value of the Laplacian change rate for each region and layer.

7. The X-ray image processing device of claim 1, wherein the inverse converter is configured to:
perform a smoothing operation for each layer based on a histogram-based flattened value for the layers corresponding to the intermediate frequency portion, the anatomy region information, and edge map information for each layer.

8. A method of processing an X-ray image, the method comprising:
receiving an original X-ray image;
analyzing the received original X-ray image;
converting the original X-ray image into a multi-scale based on the analysis results and separating the multi-scale into frequency units;
generating a noise prediction map by enhancing and combining specific frequency portions;
generating an edge map based on the generated noise prediction map;
enhancing a contrast of the X-ray image based on the generated edge map;
performing a flattening process and an edge correction process on the contrast-enhanced X-ray image and inversely converting the X-ray image; and
tone mapping and outputting the inversely converted X-ray image.

9. The X-ray image processing method of claim 8, wherein the step of analyzing the X-ray image includes:
segmenting the X-ray image into an anatomy region, a direct exposure region, and a collimation region; and
separating a noise region and an edge region for the segmented anatomy region, suppressing the separated noise region, and boosting the separated edge region.

10. The method of claim 9, wherein the conversion of the original X-ray image into multi-scale is performed by using a Gaussian-Laplacian pyramid structure.

11. The method of claim 10, wherein the step of generating the noise prediction map is performed for each layer based on information on a plurality of layers corresponding to high-frequency portions, and
wherein the information on the plurality of layers is based on Laplacian values calculated using Gaussian information corresponding to the brightness of specific frequency portions and the original image.

12. The method of claim 11, wherein the step of generating the noise prediction map corrects noise prediction information for the next higher layer by reflecting the noise prediction information predicted for the highest layer among the plurality of layers.

13. The method of claim 12, wherein the step of generating the edge map is performed in units of each layer corresponding to the high-frequency portions, and includes:
generating a contrast map; and
determining and applying whether to suppress or suppress strength for the edge map of each layer by correlating each layer corresponding to the high-frequency portions,
wherein the step of determining and applying whether to suppress or suppress strength for the edge map of each layer by correlating each layer corresponding to the high-frequency portions includes dividing a combined value of the generated contrast map and the generated edge map by the noise prediction information for the corresponding layer, based on the anatomy region information.

14. The X-ray image processing method of claim 13, further comprises:
performing a range control of normalization on each layer corresponding to the high-frequency portions based on a predefined target value of the Laplacian change rate for each region and layer.

15. The method of claim 8, wherein the step of inversely converting performs a smoothing operation for each layer based on the histogram-based flattened value for the layers corresponding to the intermediate frequency portion, the anatomy region information, and edge map information for each layer.
